# EUROPEAN PATENT SPECIFICATION

(11) **EP 3 750 575 B1**
(45) Date of publication and mention of the grant of the patent: **12.06.2024**
(21) Application number: 19760595.9
(22) Date of filing: 01.02.2019
(51) Int. Cl.: A61M 5/168, A61M 5/165, A61M 5/36, A61M 5/14, A61M 5/38

(54) **MEDICINAL FLUID SUPPLY CONTROL DEVICE AND INJECTION LIQUID INJECTION DEVICE**
STEUERVORRICHTUNG FÜR MEDIZINISCHE FLÜSSIGKEITSZUFUHR UND INJEKTIONSVORRICHTUNG FÜR INJEKTIONSFLÜSSIGKEIT
DISPOSITIF DE COMMANDE D'ALIMENTATION EN FLUIDE MÉDICINAL ET DISPOSITIF D'INJECTION DE LIQUIDE D'INJECTION

(30) Priority: 27.02.2018 KR 20180023874
(43) Date of publication of application: 16.12.2020
(73) Proprietor: E-Wha Meditech Inc., Goyang-si, Gyeonggi-do 10454 (KR)
(72) Inventor: KIM, Yong Hyun, Goyang-si Gyeonggi-do 10483 (KR)
(74) Representative: Isarpatent
(86) International application number: PCT/KR2019/001458
(87) International publication number: WO 2019/168277

(56) References cited:
- EP-A2- 3 251 708
- KR-A- 20030 089 478
- KR-A- 20100 091 100
- KR-A- 20150 029 283
- KR-A- 20160 105 115
- US-A1- 2012 053 556
- US-A1- 2012 157 918
- US-A1- 2012 157 918

## Description

### TECHNICAL FIELD

The present invention relates to a device for regulating liquid medicine supply capable of continuously supplying a certain amount of liquid medicine to a patient, and rapidly supplying an increased amount of liquid medicine depending on a patient's condition.

Specifically, the present invention relates to a device for regulating liquid medicine supply, wherein air that exists in a liquid medicine or between portions of a liquid medicine is removed in advance by an air removal filter provided on a discharge path in the device so that "priming" as a preparatory operation for injection of the liquid medicine into a patient can be accelerated and it is possible to reduce an unnecessary loss of the liquid medicine, which is caused by discharge of the liquid medicine for removal of the air existing in the liquid medicine or between portions of the liquid medicine.

Further, the present invention relates to a device for regulating liquid medicine supply, wherein air existing between portions of a liquid medicine, which may be produced by a difference in flow speeds of the portions of the liquid medicine flowing in the device, is removed by an air removal filter provided on a discharge path in the device so that an increment of the liquid medicine can be rapidly injected and a user can directly and conveniently regulate the injection amount of the liquid medicine as desired.

Additionally, the present invention relates to a liquid medicine injection apparatus including the aforesaid device for regulating liquid medicine supply.

### BACKGROUND ART

There is a case where a liquid medicine such as an antibiotic, an analgesic or an anticancer drug is slowly injected into a patient's body for a period of time. In principle, a constant amount per hour is continuously injected into the patient's body, although depending on a patient's condition, the supply amount of the liquid medicine may be temporarily increased and injected into the patient's body.

Such injection of a temporary increment of a liquid medicine, or bolus injection refers to injection of a relatively large amount of liquid medicine into a patient's body when there is a need for rapid increase in concentration of a liquid medicine such as an antibiotic, an analgesic or an anti-cancer drug in the patient's body up to a predetermined level. That is, the bolus injection is to increase the concentration of the liquid medicine in the blood so as to quickly benefit from the liquid medicine.

In general, the bolus injection may be applied when a liquid medicine such as an antibiotic, an analgesic or an anticancer drug is simply injected at a time, and may often be performed through a tube that allows the liquid medicine to be slowly injected into a patient's body for a period of time. At this time, a device installed in the middle of a tube with the liquid medicine flowing therethrough so as to variously regulate an injection amount per hour and to temporarily increase the supply amount of the liquid medicine is a liquid medicine-injecting device (referred to herein as a device for regulating liquid medicine supply).

For example, a liquid medicine-injecting device used for the bolus injection by which a certain amount of liquid medicine is injected at a time is disclosed in Korean Patent Laid-Open Publication No. 10-2001-0103116, Korean Patent Publication No. 10-0516446, and International Publication No. WO2016/137247 A2 also published as EP 3 251 708 A2. These publications disclose that in an entire liquid medicine-injecting system in which one end of a tube is connected to a liquid medicine storage container and the other end of the tube is connected to a patient's blood vessel through a needle or catheter, a liquid medicine-injecting device (a device for regulating the injection amount of a liquid medicine) is separately provided in the middle of the tube with the liquid medicine flowing therethrough. This conventional liquid medicine-injecting device (the device for regulating the injection amount of the liquid medicine) has a configuration in which when an increment of the liquid medicine is required, a liquid medicine injection button is pushed to discharge the liquid medicine stored in a liquid medicine reservoir or liquid medicine pouch so that a flow rate of the liquid medicine flowing in the tube can be increased, thereby increasing the amount of the liquid medicine to be injected into the patient's body.

However, the aforementioned prior art removes residual air in the liquid medicine-injecting device or tube by draining the liquid medicine before the tube is connected to the catheter or needle. Since a dangerous situation may occur if air enters the patient's body, air existing in the liquid medicine-injecting device or tube should be removed during the "priming" process that is a preparatory operation for injecting the liquid medicine into the patient' body. However, since it takes time to discharge an amount of liquid medicine in order to remove air in the liquid medicine or between portions of the liquid medicine, the conventional liquid medicine-injecting device makes the priming process slowed down and produces an unnecessary loss of the liquid medicine.

Moreover, as for the conventional liquid medicine-injecting device, the speed of the liquid medicine in a continuous injection flow passage for the liquid medicine is different from the speed of the liquid medicine in an increment injection flow passage for the liquid medicine. The liquid medicine flowing through the continuous injection flow passage is discharged directly through an outflow tube without passing through the liquid medicine reservoir or liquid medicine pouch, whereas the liquid medicine flowing through the increment injection flow passage is divided from the continuous injection flow passage and stored in the liquid medicine reservoir or liquid medicine pouch, and is then joined from the liquid medicine reservoir or liquid medicine pouch to the continuous injection flow passage by means of an operation of a push button. Therefore, there may be a high possibility that air exists between the liquid medicine flowing through the continuous injection flow passage and the liquid medicine flowing through the increment injection flow passage. If such air between portions of the liquid medicine enters the patient's body, the air makes a patient dangerous and also slows an injection speed of an increment of the liquid medicine in the liquid medicine-injecting device.

In order to solve the problems, an air-controlling filter cap is conventionally installed at an end of the outflow tube through which the liquid medicine exiting the liquid medicine-injecting device is discharged. However, since the air-controlling filter cap is installed at the extreme end of the outflow tube, it takes a long time for the liquid medicine with air entrained therein to reach this filter cap, and thus there is a problem that air is removed after the liquid medicine has flowed for a long time.

That is, although the liquid medicine-injecting device of Korean Patent Publication No. 10-0516446 and International Publication No. WO2016/137247 A2 as described above is an excellent technology, continuous improvement may be made thereto like other excellent technologies. For example, it is necessary to provide a novel liquid medicine-injecting device in which a process of discharging the liquid medicine for the priming in the liquid medicine-injecting device is not required, so that priming can be accelerated, an unnecessary loss of the liquid medicine can be reduced, and injection of an increment of the liquid medicine can be rapidly performed.

Therefore, the device for regulating liquid medicine supply of the present invention relates to a technology capable of accelerating "priming" that is a preparatory operation for injecting a liquid medicine into a patient, reducing an unnecessary loss of the liquid medicine, and rapidly performing the injection of an increment of the liquid medicine.

US 2012/0053556 A1 describes an infusion control device.

US 2012/0157918 A1 describes a patient controlled liquid drug administration device.

Meanwhile, it is to be understood that the matters described as the background art are intended merely to aid in the understanding of the background of the present invention and are not admitted as prior art against the present invention.

### SUMMARY

The present invention is conceived to solve the problems of the prior art described above and to provide various additional advantages. An object of the present invention is to provide a device for regulating liquid medicine supply capable of continuously supplying a certain amount of liquid medicine to a patient, and rapidly supplying an increased amount of liquid medicine depending on a patient's condition.

Specifically, an object of the present invention is to provide a device for regulating liquid medicine supply, wherein air that exists in a liquid medicine or between portions of a liquid medicine is removed in advance by an air removal filter provided on a discharge path in the device so that "priming" as a preparatory operation for injection of the liquid medicine into a patient can be accelerated and it is possible to reduce an unnecessary loss of the liquid medicine, which is caused by discharge of the liquid medicine for removal of the air existing in the liquid medicine or between portions of the liquid medicine.

Another object of the present invention is to provide a device for regulating liquid medicine supply, wherein air existing between portions of a liquid medicine, which may be produced by a difference in flow speeds of the portions of the liquid medicine flowing in the device, is removed by an air removal filter provided on a discharge path in the device so that an increment of the liquid medicine can be rapidly injected and a user can directly and conveniently regulate the injection amount of the liquid medicine as desired.

A further object of the present invention is to provide a device for regulating liquid medicine supply, wherein the injection amount of a liquid medicine can be accurately and quickly modified or regulated by changing a diameter and/or length of an internal flow passage provided in the device.

A still further object of the present invention is to provide a liquid medicine injection apparatus including the aforesaid device for regulating liquid medicine supply.

However, the objects of the present invention as described above are illustrative and the scope of the present invention is not limited thereby. In addition, other objects and advantages of the present invention will be more apparent from the following description, the appended claims and the accompanying drawings.

These objects are achieved by a device for regulating liquid medicine supply provided according to the present invention.

The present invention provides a device for regulating liquid medicine supply according to claim 1.

The device for regulating liquid medicine supply may further comprise a liquid medicine increment-controlling means which is provided in the casing to be movable between an operating position at which the increment-supplying flow passage is opened and a non-operating position at which the increment-supplying flow passage is closed and blocked, and which is shifted from the non-operating position to the operating position or from the operating position to the non-operating position so as to supply an increment of the liquid medicine stored in the increment-supplying flow passage to the liquid medicine outflow tube through the outlet end of the continuous supply flow passage or to block the supply of the increment of the liquid medicine.

In the device for regulating liquid medicine supply, even though air exists in the continuous supply flow passage and/or the increment-supplying flow passage, air, which is entrained in the liquid medicine passing through the outlet end of the continuous supply flow passage or is between portions of the liquid medicine passing through the outlet end of the continuous supply flow passage, is removed by the air removal means, and thus "priming" for injection of an increment of the liquid medicine is accelerated. When the continuous supply flow passage has been primed, air that may possibly exist is removed from the increment-supplying flow passage by the air removal means, so that the priming can be completed without waiting for the inflow of the liquid medicine from the increment-supplying flow passage. Furthermore, since the air removal means removes air entrained in the liquid medicine or between portions of the liquid medicine, it is not necessary for a physician or medical staff to waste some of the liquid medicine when injecting the liquid medicine into a patient, thereby preventing a problem of the waste of the liquid medicine and minimizing a loss of the liquid medicine.

In the device for regulating liquid medicine supply, the continuous supply flow passage may comprise a connection passage being in communication with the inflow inlet within the body, a first capillary tube passage being in communication with the connection passage, and a first internal flow passage for connecting the first capillary tube passage to the discharging outlet within the body so as to discharge the liquid medicine, which has passed through the first capillary tube passage, through the discharging outlet.

In the device for regulating liquid medicine supply, the increment-supplying flow passage may comprise an internal flow passage provided inside the body, and an external flow passage provided outside the body and being in communication with the internal flow passage.

In the device for regulating liquid medicine supply, the internal flow passage may comprise a connection passage being in communication with the inflow inlet within the body, a second capillary tube passage being in communication with the connection passage, a second internal flow passage being in communication with the second capillary tube passage, and an outlet of the second internal flow passage for guiding the liquid medicine, which has passed through the second capillary tube passage, to the external flow passage.

In the device for regulating liquid medicine supply, the external flow passage may comprise a reservoir inflow tube for connecting the outlet of the second internal flow passage to a reservoir inlet at the outside of the body, a reservoir having a liquid medicine storage space for storing the liquid medicine provided through the reservoir inflow tube, and a reservoir outflow tube for allowing the liquid medicine, which is discharged from the liquid medicine storage space of the reservoir through a reservoir outlet, to join at the first internal flow passage.

In the device for regulating liquid medicine supply, the filter member may be formed as a dual filter composed of a hydrophilic filter layer and a gas-permeable and liquid-impermeable hydrophobic filter layer stacked on the hydrophilic filter layer. The liquid medicine that has arrived earlier relative to air is absorbed by the hydrophilic filter layer having a liquid absorption power before the liquid medicine reaches the gas-permeable and liquid-impermeable hydrophobic filter layer. When all of the liquid medicine that has introduced earlier relative to air is absorbed by the hydrophilic filter layer, air that is being introduced after the absorption of the liquid medicine reaches and passes through the gas-permeable and liquid-impermeable hydrophobic filter layer and is then discharged to the outside of the body through the air vent of the cover member. Therefore, the filter member with the aforementioned configuration can effectively remove air and prevent leakage of the liquid medicine if the liquid medicine reaches the filter member earlier relative to air.

In the device for regulating liquid medicine supply, the first capillary tube passage and the second capillary tube passage may be identical to or different from each other in diameter and/or length, and the diameter and/or length thereof may be appropriately adjusted depending on a desired flow rate of the liquid medicine. Therefore, the supply amount of the liquid medicine can be regulated by changing the diameter and/or length of the first capillary tube passage or the second capillary tube passage. The change of the diameter and/or length of the first capillary tube passage or the second capillary tube passage may be achieved by replacing the first capillary tube or the second capillary tube with a capillary tube having a different diameter and/or length.

In the device for regulating liquid medicine supply, a finger insertion aperture may be provided in one end of the casing so that a user can insert a finger thereinto for manipulation. The body is disposed within the casing and below the finger insertion aperture, the reservoir is disposed above the body, and the liquid medicine increment-controlling means is disposed above the reservoir. The liquid medicine increment-controlling means has an actuation button of which one end being disposed to inwardly protrude toward a center of the finger insertion aperture, and of which the other end being disposed toward an upper side of the reservoir so as to pressurize the reservoir. Preferably, the liquid medicine increment-controlling means may further include a pressing part disposed at a lower end of the actuation button to press the reservoir whenever the actuation button is pressed.

In the device for regulating liquid medicine supply, an upper end of the actuation button may protrude toward the finger insertion aperture, and may be resiliently supported by an elastic member, e.g., a spring, within the casing. By pressing the actuation button, the liquid medicine increment-controlling means is shifted from the non-operating position to the operating position.

### ADVANTAGEOUS EFFECTS

With the configuration described above, the present invention allows air that exists in a liquid medicine or between portions of a liquid medicine to be removed in advance so that "priming" as a preparatory operation for injection of the liquid medicine into a patient can be accelerated and it is possible to reduce an unnecessary loss of the liquid medicine, which is caused by discharge of the liquid medicine for removal of the air existing in the liquid medicine or between portions of the liquid medicine. That is, according to the present invention, since the air that exists in the liquid medicine or between the portions of the liquid medicine is removed by an air removal means on a discharge path through which the liquid medicine is supplied to the liquid medicine outflow tube, the priming is accelerated; and since there is no need to discharge the liquid medicine for the priming, it is possible to reduce an unnecessary loss of the liquid medicine.

Further, according to the present invention, air existing between portions of a liquid medicine, which may be produced by a difference in flow speeds of the portions of the liquid medicine flowing in the device for regulating liquid medicine supply, is removed by an air removal means provided on a discharge path in the device so that an increment of the liquid medicine can be rapidly injected and a user can directly and conveniently regulate the injection amount of the liquid medicine as desired.

It should be understood that the scope of the present invention is not limited to the aforementioned effects.

### BRIEF DESCRIPTION OF DRAWINGS

FIG. 1 is an external perspective view showing a state where a user grips a device for regulating liquid medicine supply 200 according to one embodiment of the present invention.
FIG. 2 is a front view showing arrangement of components in a casing 201 in a state where the device for regulating liquid medicine supply 200 according to the embodiment of the present invention is disassembled.
FIG. 3 is a side view of a body 210 of the device for regulating liquid medicine supply 200 according to the embodiment of the present invention.
FIG. 4 is a perspective view showing an interrelationship between the body 210 and a reservoir 300 in the device for regulating liquid medicine supply 200 according to the embodiment of the present invention.
FIG. 5 is an exploded perspective view of the body 210 of the device for regulating liquid medicine supply 200 according to the embodiment of the present invention.
FIG. 6 is a longitudinal sectional view of the body 210 of the device for regulating liquid medicine supply 200 according to the embodiment of the present invention, taken along line "A-A" of FIG. 3.
FIG. 7 is a sectional view taken along line "B-B" of FIG. 6.
FIG. 8 is an enlarged view of "C" portion of FIG. 7, showing an air removal means.
FIG. 9 is a view showing a configuration of one example of a liquid medicine injection apparatus 100 including the device for regulating liquid medicine supply 200 according to the embodiment of the present invention.

### DETAILED DESCRIPTION

Hereinafter, preferred embodiments according to the present invention will be described in detail with reference to the accompanying drawings. The following embodiments of the present invention are just to implement the present invention and are not intended to limit or restrict the scope of the present invention. Thus, those that can be easily contemplated by persons skilled in the art from the detailed description and examples of the present invention are interpreted to fall within the scope of the present invention.

Throughout the present specification, it is to be understood that, when any part is referred to as "comprising" any component, it does not exclude other components, but may further include other components, unless otherwise specified.

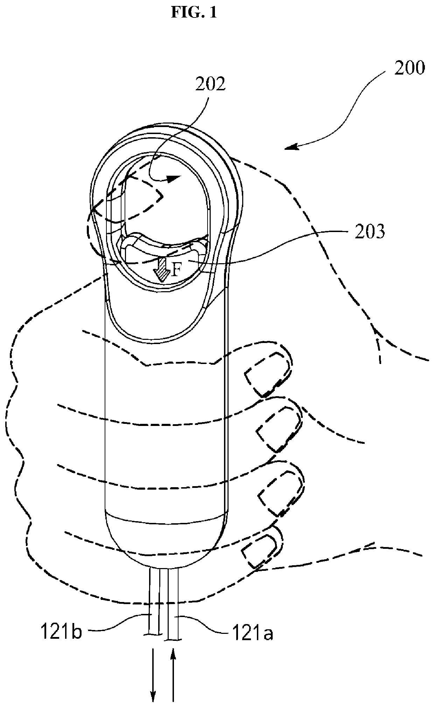

FIG. 1 is an external perspective view showing a state where a user grips a device for regulating liquid medicine supply 200 according to one embodiment of the present invention. As shown in FIG. 1, a liquid medicine flows into the device for regulating liquid medicine supply 200 from a liquid medicine inflow tube 121a connected to a liquid medicine storage space (not shown), and the liquid medicine which has flowed into the device for regulating liquid medicine supply 200 is discharged to a liquid medicine outflow tube 121b. For reference, the device in a longitudinally extending form as shown in FIG. 1 is merely one example, and it will be apparent that a body 210, a continuous supply flow passage CL, increment-supplying flow passages VL1 and VL2, a liquid medicine increment-controlling means 220, a reservoir 300, air removal means 224, 225, 225a and 226 and the like, which are main components of the present invention as described below, may be applied to cases having various forms and also applicable to a form disclosed in Korean Patent Publication No. 10-0516446, for example.

In general, a liquid medicine such as an anticancer drug, an antibiotic, an analgesic or the like is stored in the liquid medicine storage space. For example, various kinds of injectable drug-pumping devices such as a mechanical injection device in which a plunger of a syringe is moved forward slowly by a driving force of a motor so as to push out the liquid medicine filled in the liquid medicine storage space, a balloon type injection device in which the liquid medicine filled in the liquid medicine storage space is slowly pushed out by an elastic restoring force of a balloon, or a gas-generating type injection device (see Korean Patent Publication No. 10-0507593) in which a gas at a certain pressure slowly moves a piston forward so as to push out the liquid medicine filled in the liquid fluid storage space, and the like may be connected to the device for regulating liquid medicine supply 200 through the liquid medicine inflow tube 121a.

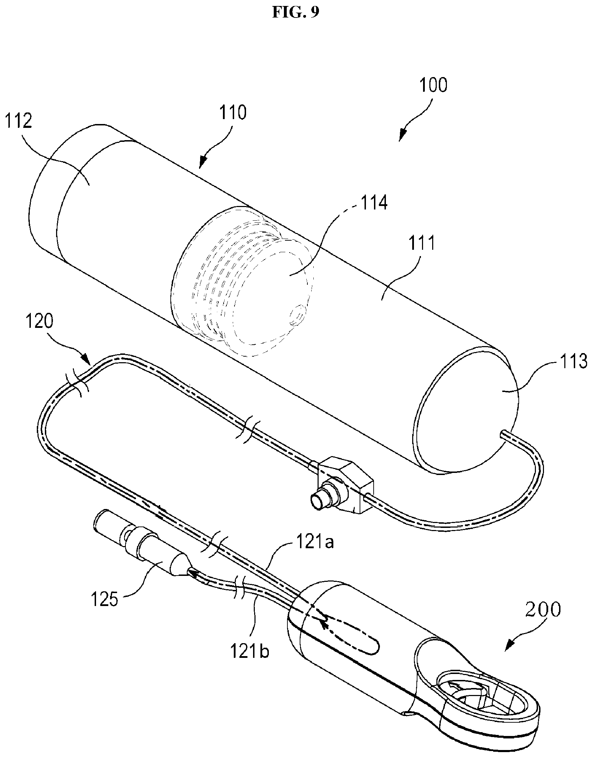

As an example which does not limit the present invention, FIG. 9 schematically shows an overall configuration of a liquid medicine injection apparatus 100 including the device for regulating liquid medicine supply 200 of the embodiment of the present invention. In FIG. 9, reference numeral 100 generally denotes the liquid medicine injection apparatus, reference numeral 110 denotes a gas-generating type injection device which is an example of an injectable drug-pumping device, and reference numeral 200 denotes the device for regulating liquid medicine supply. As one example, the liquid medicine injection apparatus 100 includes the gas-generating type injection device 110 and the device for regulating liquid medicine supply 200. The device for regulating liquid medicine supply 200 connected to the gas-generating type injection device 110 through a conduit 120 and the liquid medicine inflow tube 121a regulates the amount of the liquid medicine supplied from the gas-generating type injection device 110 through the conduit 120 and the liquid medicine inflow tube 121a, and then supplies the liquid medicine to a catheter or injection needle (not shown) connected to an end cap 125 provided at an end of the liquid medicine outflow tube 121b. In the gas-generating type injection device 110, a piston 114 is moved forward by a pressure of a gas generated in a gas generating part 112, and as the piston 114 is moved forward, the liquid medicine in a liquid medicine storage space 111 is supplied into the device for regulating liquid medicine supply 200 through the conduit 120 connected to a cylinder tip 113 and the liquid medicine inflow tube 121a. However, it will be apparent that instead of the aforementioned gas-generating type injection device 110, various kinds of injectable drug-pumping devices, such as a mechanical injection device, a balloon type injection device, or the like may be used to be connected to the device for regulating liquid medicine supply 200.

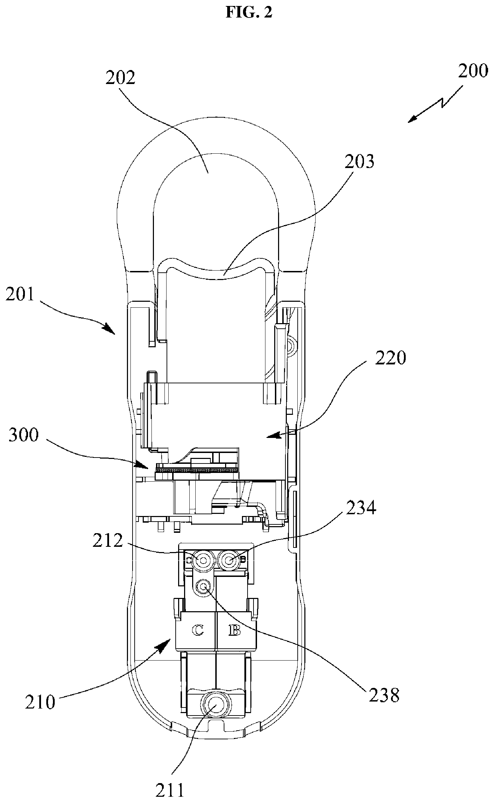

FIG. 2 is a front view showing arrangement of components in a casing in the device for regulating liquid medicine supply 200 according to the embodiment of the present invention. As shown in FIG. 2, the device for regulating liquid medicine supply 200 has a casing 201; the body 210 provided inside a lower part of the casing 201 so that the liquid medicine supplied from the injectable drug-pumping device flows into the body through an inflow inlet 211, is regulated to a predetermined amount in the body, and is then discharged through a discharging outlet 212; and a liquid medicine increment-controlling means 220 for increasing the amount of the liquid medicine flowing outwardly through the discharging outlet 212 of the body 210, as desired.

Here, the casing 201 may be constructed of a front casing and a rear casing which are divided in half along a longitudinal direction, and the front casing may be detachably assembled to the rear casing. In addition, a finger insertion aperture 202 is formed at an upper portion of the casing 201 such that a user can insert a finger thereinto to perform an operation of pressing an actuation button 203.

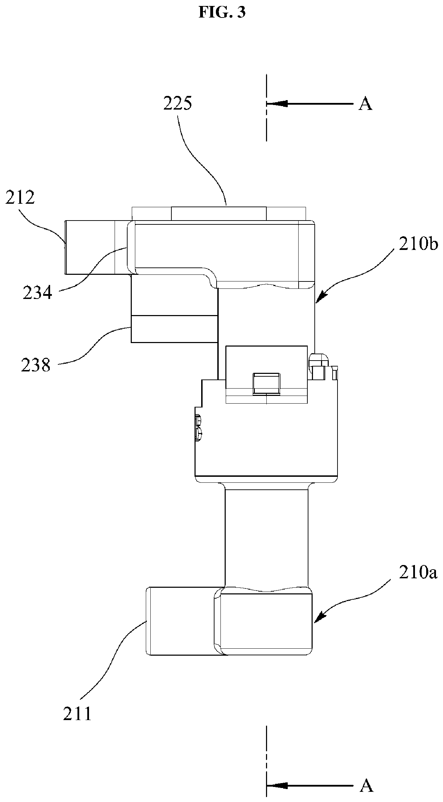

The body 210 is provided at a lower portion of the casing 201. A configuration of the body 210 is shown in FIGS. 3 to 7.

The body 210 controls the liquid medicine, which has flowed from the injectable drug-pumping device through the inflow inlet 211 into the body, to a predetermined amount and supplies the liquid medicine to the discharging outlet 212 and the liquid medicine outflow tube 121b. Optionally, the body 210 may further supply the liquid medicine, which has been stored in the reservoir 300, to the discharging outlet 212 by means of an operation of the liquid medicine increment-controlling means 220, thereby discharging an increased amount of liquid medicine through the discharging outlet 212 and the liquid medicine outflow tube 121b.

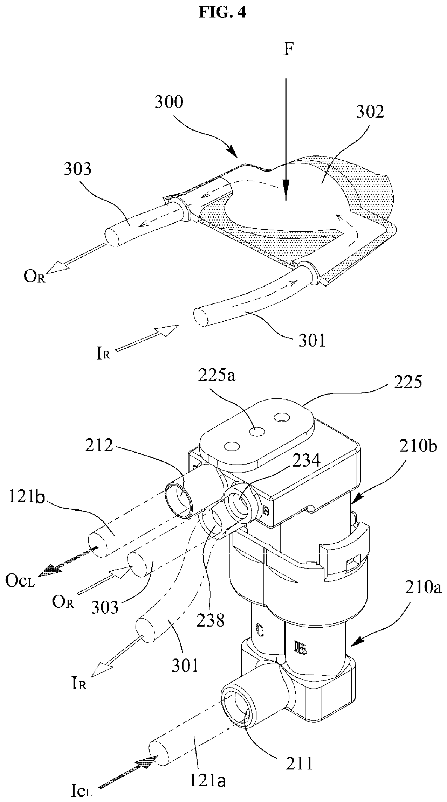

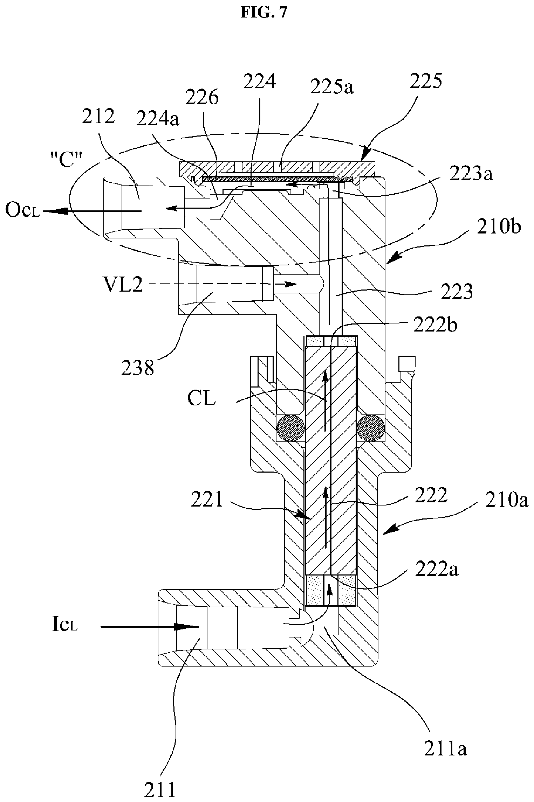

A continuous supply flow passage CL, configured to regulate a flow rate of the liquid medicine that has flowed through the inflow inlet 211 and to continuously supply a certain amount of liquid medicine to the liquid medicine outflow tube 121b through the discharging outlet 212, is provided within the body 210 (see FIGS. 4, 6 and 7). As shown in FIGS. 4 and 7, the liquid medicine flows into the body 210 in an "I_{CL}" direction, and is discharged out of the body 210 in an "O_{CL}" direction.

In addition, the liquid medicine increment-controlling means 220 has the increment-supplying flow passages VL1 and VL2 which divide and store the liquid medicine flowing into the body 210 through the inflow inlet 211 of the body 210 and then selectively allow the divided and stored liquid medicine to join at an outlet end of the continuous supply flow passage CL so as to selectively increase the amount of the liquid medicine discharged through the discharging outlet 212 (see FIGS. 4, 6 and 7).

In one embodiment of the present invention, the body 210 preferably consists of a lower body 210a and an upper body 210b, and the upper body 210b is coupled to an upper end of the lower body 210a. The inflow inlet 211 is provided in the lower body 210a, and the discharging outlet 212 is provided in the upper body 210b. A leading end of the liquid medicine inflow tube 121a for guiding the liquid medicine from the injectable drug-pumping device is connected to the inflow inlet 211, and the liquid medicine outflow tube 121b for guiding the liquid medicine to the end cap 125 is connected to the discharging outlet 212.

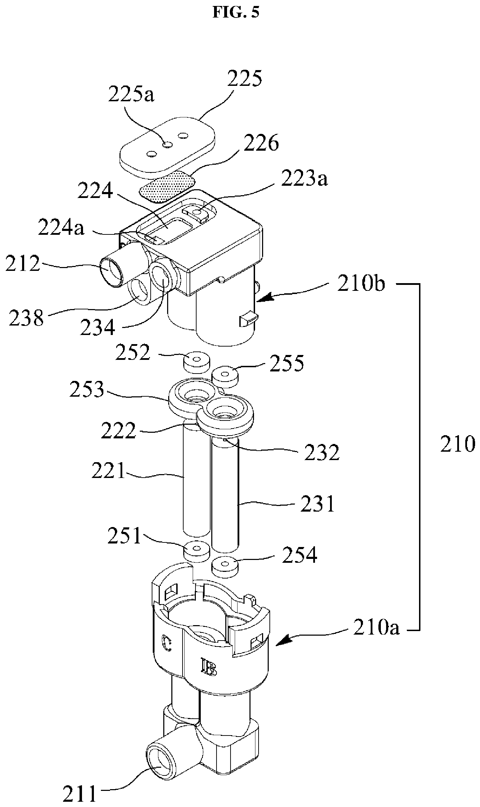

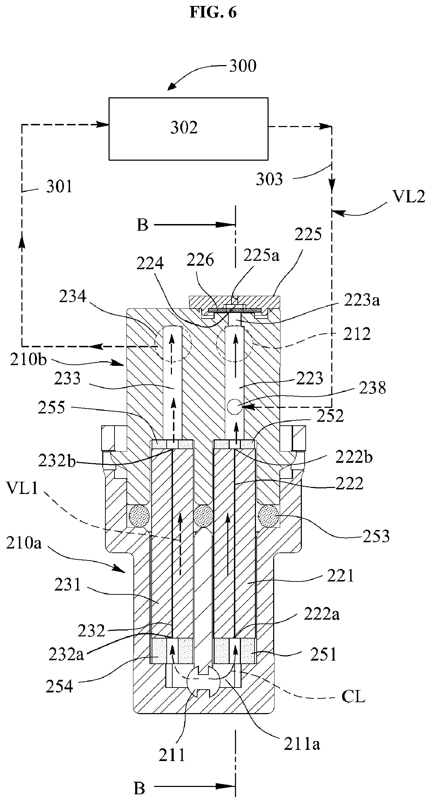

As shown in FIGS. 5 and 6, a first capillary tube 221 is inserted into the body 210, and a first capillary tube passage 222 of the first capillary tube 221 introduces the liquid medicine, which has flowed thereinto from the inflow inlet 211, into a first internal flow passage 223 due to a capillary phenomenon. At this time, the liquid medicine flows into the first capillary tube passage 222 through an inlet 222a of the first capillary tube 221 and is then regulated to a certain flow rate by passing through the first capillary tube passage 222. The liquid medicine regulated to the certain flow rate escapes from an outlet 222b of the first capillary tube passage 222 and is then introduced into the first internal flow passage 223. The liquid medicine, which has been regulated to the certain flow rate by passing through the first capillary tube passage 222 and the first internal flow passage 223, is discharged out of the upper body 210b through the discharging outlet 212, and is then supplied to the end cap 125 through the liquid medicine outflow tube 121b.

The first capillary tube 221 is fitted into a hole of an approximately "O"-shaped ring 253, and the "O"-shaped ring 253 into which the first capillary tube 221 has been fitted is snugly fitted into an empty space between the lower body 210a and the upper body 210b to be coupled to the bodies (see FIGS. 5 and 6). Furthermore, a first sealing ring 251 is fitted between the inlet 222a of the first capillary tube 221 and the lower body 210a to be coupled thereto and a second sealing ring 252 is fitted between the outlet 222b of the first capillary tube 221 and the upper body 210b to be coupled thereto, thereby preventing the liquid medicine from leaking from a junction between the first capillary tube 221 and the lower body 210a and a junction between the first capillary tube 221 and the upper body 210b.

As shown in FIGS. 6 and 7, the inflow inlet 211, a connection passage 211a, the first capillary tube passage 222, the first internal flow passage 223, an outlet end 223a of the first internal flow passage, a groove 224, an outlet 224a, and the discharging outlet 212 are sequentially connected to one another to form the continuous supply flow passage CL.

Moreover, the embodiment of the present invention includes a liquid medicine increment means which can selectively increase the flow rate of the liquid medicine supplied to the liquid medicine outflow tube 121b through the discharging outlet 212 after the liquid medicine has passed through the continuous supply flow passage CL, if necessary.

The liquid medicine increment means of the embodiment of the present invention may include the increment-supplying flow passages VL1 and VL2 which divide and store the liquid medicine flowing into the body 210 through the inflow inlet 211 of the body 210 and then selectively allow the dived and stored liquid medicine to join at the outlet end of the continuous supply flow passage CL so as to selectively increase the amount of the liquid medicine discharged through the discharging outlet 212; and the liquid medicine increment-controlling means 220 which is provided in the casing 201 to be movable between an operating position at which the increment-supplying flow passages VL1 and VL2 are opened and a non-operating position at which the increment-supplying flow passages VL1 and VL2 are closed and blocked, and which is shifted from the non-operating position to the operating position or from the operating position to the non-operating position so as to supply an increment of the liquid medicine stored in the increment-supplying flow passages VL1 and VL2 to the liquid medicine outflow tube 121b through the outlet end of the continuous supply flow passage CL or to block the supply of the increment of the liquid medicine.

As shown in FIG. 6, the increment-supplying flow passage VL includes an internal flow passage VL1 provided inside the body 210, and an external flow passage VL2 provided outside the body 210 and being in communication with the internal flow passage VL1. As shown in FIG. 4, the reservoir 300 is connected to the external flow passage VL2. Referring to FIG. 4, the liquid medicine flows into the reservoir 300 from the body 210 (specifically, an outlet 234 of a second internal flow passage) through a reservoir inflow tube 301 in an "I_{R}" direction and is then stored in a liquid medicine storage space 302, and the liquid medicine in the liquid medicine storage space 302 flows into the body 210 (specifically, an inlet 238 of the first internal flow passage) through a reservoir outflow tube 303 in an "O_{R}" direction.

As shown in FIGS. 5 and 6, the internal flow passage VL1 includes the connection passage 211a which is in communication with the inflow inlet 211 in the body 210, a second capillary tube passage 232 which is in communication with the connection passage 211a, the second internal flow passage 233 which is in communication with the second capillary tube passage 232, and the outlet 234 of the second internal flow passage for guiding the liquid medicine, which has passed through the second capillary tube passage 232, to the external flow passage VL2. A second capillary tube 231 is inserted into the body 210, and the second capillary tube passage 232 of the second capillary tube 231 guides the liquid medicine, which has flowed from the inflow inlet 211, to the second internal flow passage 233 due to the capillary phenomenon. At this time, the liquid medicine flows into the second capillary tube passage 232 through an inlet 232a and is regulated to the certain flow rate by passing through the second capillary tube passage 232. The liquid medicine regulated to the certain flow rate escapes through an outlet 232b of the second capillary tube passage 232 and is then introduced into the second internal flow passage 233. The liquid medicine, which has been regulated to the certain flow rate by passing through the second capillary tube passage 232 and the second internal flow passage 233, is introduced into the external flow passage VL2 through the outlet 234 of the second internal flow passage.

The second capillary tube 231 is fitted into a hole of the approximately "O"-shaped ring 253, and the "O"-shaped ring 253 into which the second capillary tube 231 has been fitted is snugly fitted into the empty space between the lower body 210a and the upper body 210b to be coupled to the bodies (see FIGS. 5 and 6). Moreover, a third sealing ring 254 is fitted between the inlet 232a of the second capillary tube 231 and the lower body 210a to be coupled thereto and a fourth sealing ring 255 is fitted between the outlet 232b of the second capillary tube 231 and the upper body 210b to be coupled thereto, thereby preventing the liquid medicine from leaking from a junction between the second capillary tube 231 and the lower body 210a and a junction between the second capillary tube 231 and the upper body 210b.

As shown in FIGS. 4 and 6, the external flow passage VL2 includes, outside of the body 210, the reservoir inflow tube 301 for connecting the outlet 234 of the second internal flow passage 233 of the internal flow passage VL1 to a reservoir inlet; the reservoir 300 having the liquid medicine storage space 302 for storing the liquid medicine supplied through the reservoir inflow tube 301; and the reservoir outflow tube 303 for allowing the liquid medicine, which has been discharged from the liquid medicine storage space 302 of the reservoir 300 through a reservoir outlet, to join at the first internal flow passage 223 through the inlet 238 of the first internal flow passage.

As shown in FIGS. 1, 2 and 6, when the liquid medicine increment-controlling means 220 is operated, the actuation button 203 can press the liquid medicine storage space 302 of the reservoir 300 so as to allow the liquid medicine stored in the liquid medicine storage space 302 to join at the first internal flow passage 223 through the reservoir outflow tube 303, thereby increasing the amount of the liquid medicine discharged from the discharging outlet 212.

As shown in FIG. 2, the finger insertion aperture 202 may be provided in one end of the casing 201 so that a user can insert a finger thereinto for manipulation. The body 210 is disposed within the casing 201 and below the finger insertion aperture 202, the reservoir 300 is disposed above the body 210, and the liquid medicine increment-controlling means 220 is disposed above the reservoir 300. The liquid medicine increment-controlling means 220 has the actuation button 203 of which one end being disposed to inwardly protrude toward a center of the finger insertion aperture 202, and of which the other end being disposed toward an upper side of the reservoir 300 so as to pressurize the reservoir 300. Preferably, the liquid medicine increment-controlling means 220 may further include a pressing part disposed at a lower end of the actuation button 203 to press the reservoir 300 whenever the actuation button 203 is pressed.

An upper end of the actuation button 203 may protrude toward the finger insertion aperture 202, and may be resiliently supported by an elastic member, e.g., a spring, within the casing 201. By pressing the actuation button 203, the liquid medicine increment-controlling means 220 is shifted from the non-operating position to the operating position. That is, when the user inserts a finger into the finger insertion aperture 202 of the device for regulating liquid medicine supply 200 and presses the actuation button 203 in an "F" direction (see FIG. 1), the liquid medicine increment-controlling means 220 is shifted from the non-operating position to the operating position to perform the injection of the increment of the liquid medicine.

For example, a coil spring is installed between a side wall provided at a lower end of the liquid medicine increment-controlling means 220 and a rotatable lever, wherein the coil spring applies an elastic restoring force to the lever with respect to the fixed side wall, and the lever to which the elastic restoring force of the coil spring is applied is rotated at a predetermined angle (for example, in a counterclockwise direction) and then presses the reservoir outflow tube 303. The lever to which the elastic restoring force of the coil spring is transmitted presses the reservoir outflow tube 303 to block the flow of the liquid medicine from the liquid medicine storage space 302. When the user presses the actuation button 203, a pressing force of the actuation button 203 overcomes the elastic restoring force of the coil spring, which is pressing the lever, and causes the lever to rotate in an opposite direction (for example, in a clockwise direction) so as to lift the lever from the reservoir outflow tube 303. At this time, the blockage of the reservoir outflow tube 303 is released, and at the same time, the pressing part of the actuation button 203 presses the liquid medicine storage space 302, so that the liquid medicine in the liquid medicine storage space 302 flows in the "O_{R}" direction (see FIG. 4). Mutual coupling relationships, operations and functions of the actuation button 203, the liquid medicine increment-controlling means 220 and the reservoir 300 are the same as those described in International Publication No. WO 2016/137247 A2.

Meanwhile, since the actuation button 203, the liquid medicine increment-controlling means 220, the reservoir 300, various tubes and coupling relationships thereof, and other components in the device for regulating liquid medicine supply 200 of the embodiment of the present invention are described in International Publication WO 2016/137247 A2 and those skilled in the art can easily apply and adapt them to the present invention, detailed descriptions of these components will be omitted.

In addition, the first capillary tube passage 222 and the second capillary tube passage 232 may be identical to or different from each other in diameter and/or length, and the diameter and/or length thereof may be appropriately adjusted depending on a desired flow rate of the liquid medicine. Therefore, the supply amount of the liquid medicine can be regulated by changing the diameter and/or length of the first capillary tube passage 222 or the second capillary tube passage 232. The change of the diameter and/or length of the first capillary tube passage 222 or the second capillary tube passage 232 may be achieved by replacing the first capillary tube 221 or the second capillary tube 231 with a capillary tube having a different diameter and/or length.

In order to remove air that may exist in the liquid medicine or between portions of the liquid medicine, to accelerate "priming" which is a preparatory operation for injection of the liquid medicine into a patient, and to prevent an unnecessary loss of the liquid medicine, there is provided the air removal means configured to outwardly discharge air entrained in the liquid medicine or air between portions of the liquid medicine, on a discharge path 223a, 224 and 224a which discharges the liquid medicine through the discharging outlet 212. The air removal means is provided between the outlet end of the continuous supply flow passage CL and the discharging outlet 212 of the body 210.

As shown in FIGS. 4 to 8, the air removal means includes the groove 224 concavely formed on one side of the body 210 so as to allow a portion of the continuous supply flow passage CL to be in communication with the outside of the body 210, a cover member 225 coupled to an opening portion of the groove 224 so as to seal the groove 224, at least one air vent 225a formed in the cover member 225 so as to outwardly discharge air from the liquid medicine passing through the groove 224 in the continuous supply flow passage CL, and a filter member 226 accommodated between the groove 224 and the cover member 225 so as to allow air in the liquid medicine introduced into the groove 224 to pass through the filter member and to be discharged through the air vent 225a and not to allow the liquid medicine to pass through the filter member, thereby preventing leakage of the liquid medicine.

In the device for regulating liquid medicine supply 200 of the embodiment of the present invention, even though air exists in the continuous supply flow passage CL and/or the increment-supplying flow passages VL1 and VL2, air, which is entrained in the liquid medicine passing through the outlet end of the continuous supply flow passage CL or is between portions of the liquid medicine passing through the outlet end of the continuous supply flow passage CL, is removed by the air removal means, and thus the "priming" for injection of an increment of the liquid medicine is accelerated. When the continuous supply flow passage CL has been primed, air that may possibly exist is removed from the increment-supplying flow passages VL1 and VL2 by the air removal means, so that the priming may be completed without waiting for the inflow of the liquid medicine from the increment-supplying flow passages VL1 and VL2. Furthermore, since the air removal means removes air entrained in the liquid medicine or between portions of the liquid medicine, it is not necessary for a physician or medical staff to waste some of the liquid medicine when injecting the liquid medicine into a patient, thereby preventing a problem of the waste of the liquid medicine and minimizing a loss of the liquid medicine.

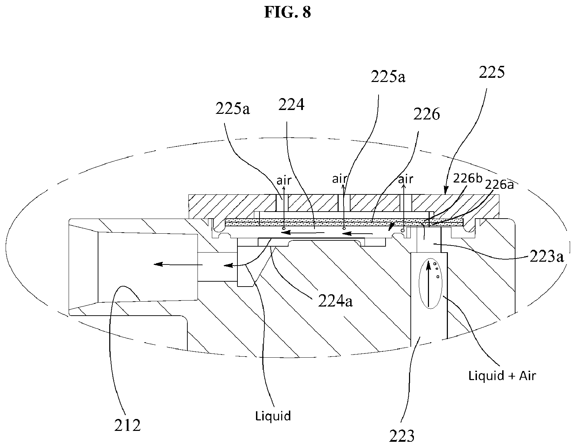

Moreover, the filter member 226 may be comprised of a single filter but may be formed as a dual filter composed of a hydrophilic filter layer 226a and a gas-permeable and liquid-impermeable hydrophobic filter layer 226b stacked on the hydrophilic filter layer 226a (See FIG. 8). The liquid medicine that has arrived earlier relative to air is absorbed by the hydrophilic filter layer 226a having a liquid absorption power before the liquid medicine reaches the gas-permeable and liquid-impermeable hydrophobic filter layer 226b. When all of the liquid medicine that has introduced earlier relative to air is absorbed by the hydrophilic filter layer 226a, air that is being introduced after the absorption of the liquid medicine reaches and passes through the gas-permeable and liquid-impermeable hydrophobic filter layer 226b and is then discharged to the outside of the body 210 through the air vent 225a of the cover member 225. Therefore, the filter member 226 with the aforementioned configuration can effectively remove air and prevent leakage of the liquid medicine if the liquid medicine reaches the filter member earlier relative to air.

Although the present invention has been described with reference to the embodiments, the scope of the present invention is not limited to these embodiments. Any person skilled in the art will appreciate that various modifications and changes are possible without departing from the scope of the present invention and these modifications and changes also fall within the scope of the present invention.

## Claims

1. A device for regulating liquid medicine supply (200), comprising:
a casing (201);
a body (210) disposed within the casing (201), and having an inflow inlet (211) through which a liquid medicine from a liquid medicine inflow tube (121a) extending to be in communication with a liquid medicine storage space flows into the body (210), and a discharging outlet (212) for discharging the liquid medicine, which has flowed into the body (210) through the inflow inlet (211), to a liquid medicine outflow tube (121b);
a continuous supply flow passage (CL) provided inside the body (210) and configured to regulate a flow rate of the liquid medicine flowing into the body (210) through the inflow inlet (211) so that a certain amount of liquid medicine is continuously supplied to the liquid medicine outflow tube (121b) through the discharging outlet (212) of the body (210);
an increment-supplying flow passage (VL1, VL2) configured to divide and store the liquid medicine flowing into the body (210) through the inflow inlet (211) of the body (210) and then to allow the divided and stored liquid medicine to join at an outlet end of the continuous supply flow passage (CL) so as to increase the amount of the liquid medicine discharged through the discharging outlet (212) of the body (210); and
an air removal means (224, 225, 225a, 226) provided between the outlet end of the continuous supply flow passage (CL) and the discharging outlet (212) of the body (210) and configured to discharge air, which is entrained in the liquid medicine passing through the outlet end of the continuous supply flow passage (CL) or is between portions of the liquid medicine passing through the outlet end of the continuous supply flow passage (CL), to the outside of the body (210) so as to remove the air,
**characterized in that** the air removal means (224, 225, 225a, 226) comprises a groove (224) concavely formed on one side of the body (210) so as to allow a portion of the continuous supply flow passage (CL) to be in communication with the outside of the body (210), a cover member (225) coupled to an opening portion of the groove (224) so as to seal the groove (224), at least one air vent (225a) formed in the cover member (225) so as to outwardly discharge air from the liquid medicine passing through the groove (224) in the continuous supply flow passage (CL), and a filter member (226) accommodated between the groove (224) and the cover member (225) so as to allow the air in the liquid medicine introduced into the groove (224) to pass through the filter member (226) and to be discharged through the air vent (225a) and not to allow the liquid medicine to pass through the filter member (226), thereby preventing leakage of the liquid medicine.

2. The device (200) of Claim 1, wherein the continuous supply flow passage (CL) comprises a connection passage (211a) being in communication with the inflow inlet (211) within the body (210), a first capillary tube passage (222) being in communication with the connection passage (211a), and a first internal flow passage (223) for connecting the first capillary tube passage (222) to the discharging outlet (212) within the body (210) so as to discharge the liquid medicine, which has passed through the first capillary tube passage (222), through the discharging outlet (212).

3. The device (200) of Claim 2, wherein the increment-supplying flow passage (VL1, VL2) comprises an internal flow passage (VL1) provided inside the body (210), and an external flow passage (VL2) provided outside the body (210) and being in communication with the internal flow passage (VL1).

4. The device (200) of Claim 3, wherein the internal flow passage (VL1) comprises a connection passage (211a) being in communication with the inflow inlet (211) within the body (210), a second capillary tube passage (232) being in communication with the connection passage (211a), a second internal flow passage (233) being in communication with the second capillary tube passage (232), and an outlet (234) of the second internal flow passage (233) for guiding the liquid medicine, which has passed through the second capillary tube passage (232), to the external flow passage (VL2).

5. The device (200) of Claim 4, wherein the external flow passage (VL2) comprises a reservoir inflow tube (301) for connecting the outlet (234) of the second internal flow passage (233) to a reservoir inlet at the outside of the body (210), a reservoir (300) having a liquid medicine storage space (302) for storing the liquid medicine provided through the reservoir inflow tube (301), and a reservoir outflow tube (303) for allowing the liquid medicine, which is discharged from the liquid medicine storage space (302) of the reservoir (300) through a reservoir outlet, to join at the first internal flow passage (223).

6. The device (200) of Claim 1, wherein the filter member (226) is formed as a dual filter comprising a hydrophilic filter layer (226a) and a gas-permeable and liquid-impermeable hydrophobic filter layer (226b) stacked on the hydrophilic filter layer (226a).

7. The device (200) of Claim 4 or 5, wherein the first capillary tube passage (222) and the second capillary tube passage (232) are identical to or different from each other in at least one of diameter or length, and the at least one of the diameter or the length of the first capillary tube passage (222) or the second capillary tube passage (232) is adjusted to regulate the supply amount of the liquid medicine.

8. A liquid medicine injection apparatus (100) comprising the device (200) of any one of Claims 1 to 5.

## Patentansprüche

1. Vorrichtung (200) zum Regulieren der Zufuhr von flüssigen Medikamenten, umfassend:
ein Gehäuse (201);
einen Körper (210), der innerhalb des Gehäuses (201) angeordnet ist und einen Zulaufeinlass (211) aufweist, durch den ein flüssiges Medikament aus einem Flüssigmedikament-Zulaufrohr (121a), das sich so erstreckt, dass es mit einem Flüssigmedikament-Speicherraum kommuniziert, in den Körper (210) fließt, und einen Abgabeauslass (212) zum Abgeben des flüssigen Medikaments, das durch den Zulaufeinlass (211) in den Körper (210) geflossen ist, zu einem Flüssigmedikament-Ausströmrohr (121b);
einen Dauerzufuhr-Strömungsdurchgang (CL), der im Inneren des Körpers (210) angeordnet und dafür eingerichtet ist, eine Strömungsrate des durch den Zulaufeinlass (211) in den Körper (210) fließenden flüssigen Medikaments so zu regulieren, dass eine bestimmte Menge des flüssigen Medikaments kontinuierlich dem Flüssigmedikament-Ausströmrohr (121b) durch den Abgabeauslass (212) des Körpers (210) zugeführt wird;
einen Inkrementzufuhr-Strömungsdurchgang (VL1, VL2), der dafür eingerichtet ist, das durch den Zulaufeinlass (211) des Körpers (210) in den Körper (210) fließende flüssige Medikament aufzuteilen und zu speichern und dann das aufgeteilte und gespeicherte flüssige Medikament an einem Auslassende des Dauerzufuhr-Strömungsdurchgangs (CL) wieder zusammenfließen zu lassen, um eine Menge des durch den Abgabeauslass (212) des Körpers (210) abgegebenen flüssigen Medikaments zu erhöhen; und
ein Luftabzugsmittel (224, 225, 225a, 226), das zwischen dem Auslassende des Dauerzufuhr-Strömungsdurchgangs (CL) und dem Abgabeauslass (212) des Körpers (210) angeordnet ist und dafür eingerichtet ist, Luft, die in dem flüssigen Medikament, das das Auslassende des Dauerzufuhr-Strömungsdurchgangs (CL) passiert, mitgeführt wird oder sich zwischen Abschnitten des flüssigen Medikaments, welches das Auslassende des Dauerzufuhr-Strömungsdurchgangs (CL) passiert, befindet, nach außerhalb des Körpers (210) abzuziehen, um die Luft zu entfernen,
**dadurch gekennzeichnet, dass** das Luftabzugsmittel (224, 225, 225a, 226) umfasst: eine Nut (224), die auf einer Seite des Körpers (210) konkav so ausgebildet ist, dass ein Abschnitt des Dauerzufuhr-Strömungsdurchgangs (CL) mit der Außenseite des Körpers (210) kommunizieren kann, ein Abdeckelement (225), das mit einem Öffnungsabschnitt der Nut (224) so gekoppelt ist, dass die Nut (224) abgedichtet wird, mindestens eine Entlüftung (225a), die in dem Abdeckelement (225) so ausgebildet ist, dass Luft aus dem durch die Nut (224) in dem Dauerzufuhr-Strömungsdurchgang (CL) strömenden flüssigen Medikament nach draußen entweichen kann, und ein Filterelement (226), das zwischen der Nut (224) und dem Abdeckelement (225) so aufgenommen ist, dass die Luft in dem in die Nut (224) eingeleiteten flüssigen Medikament das Filterelement (226) passieren und durch die Entlüftung (225a) abgelassen werden kann und das flüssige Medikament nicht das Filterelement (226) passieren kann, wodurch eine Leckage des flüssigen Medikaments verhindert wird.

2. Vorrichtung (200) nach Anspruch 1, wobei der Dauerzufuhr-Strömungsdurchgang (CL) umfasst: einen Verbindungsdurchgang (211a), der mit dem Zulaufeinlass (211) innerhalb des Körpers (210) kommuniziert, einen ersten Kapillarrohrdurchgang (222), der mit dem Verbindungsdurchgang (211a) kommuniziert, und einen ersten internen Strömungsdurchgang (223) zum Verbinden des ersten Kapillarrohrdurchgangs (222) mit dem Abgabeauslass (212) innerhalb des Körpers (210), um das flüssige Medikament, das den ersten Kapillarrohrdurchgang (222) passiert ist, durch den Abgabeauslass (212) abzugeben.

3. Vorrichtung (200) nach Anspruch 2, wobei der Inkrementzufuhr-Strömungsdurchgang (VL1, VL2) einen internen Strömungsdurchgang (VL1), der innerhalb des Körpers (210) ausgebildet ist, und einen externen Strömungsdurchgang (VL2), der außerhalb des Körpers (210) ausgebildet ist und mit dem internen Strömungsdurchgang (VL1) kommuniziert, umfasst.

4. Vorrichtung (200) nach Anspruch 3, wobei der interne Strömungsdurchgang (VL1) umfasst: einen Verbindungsdurchgang (211a), der mit dem Zulaufeinlass (211) innerhalb des Körpers (210) kommuniziert, einen zweiten Kapillarrohrdurchgang (232), der mit dem Verbindungsdurchgang (211a) kommuniziert, einen zweiten internen Strömungsdurchgang (233), der mit dem zweiten Kapillarrohrdurchgang (232) kommuniziert, und einen Auslass (234) des zweiten internen Strömungsdurchgangs (233) zum Leiten des flüssigen Medikaments, das den zweiten Kapillarrohrdurchgang (232) passiert hat, zu dem externen Strömungsdurchgang (VL2).

5. Vorrichtung (200) nach Anspruch 4, wobei der externe Strömungsdurchgang (VL2) umfasst: ein Reservoir-Zulaufrohr (301) zum Verbinden des Auslasses (234) des zweiten internen Strömungsdurchgangs (233) mit einem Reservoireinlass an der Außenseite des Körpers (210), ein Reservoir (300) mit einem Flüssigmedizin-Speicherraum (302) zum Speichern der flüssigen Medizin, die durch das Reservoir-Zulaufrohr (301) bereitgestellt wird, und ein Reservoir-Ausströmrohr (303), damit die flüssige Medizin, die aus dem Flüssigmedizin-Speicherraum (302) des Reservoirs (300) durch einen Reservoirauslass ausgelassen wird, an dem ersten internen Strömungsdurchgang (223) zusammenfließen kann.

6. Vorrichtung (200) nach Anspruch 1, wobei das Filterelement (226) als ein Doppelfilter ausgebildet ist, der eine hydrophile Filterschicht (226a) und eine gasdurchlässige und flüssigkeitsundurchlässige hydrophobe Filterschicht (226b), die auf die hydrophile Filterschicht (226a) gestapelt ist, umfasst.

7. Vorrichtung (200) nach Anspruch 4 oder 5, wobei der erste Kapillarrohrdurchgang (222) und der zweite Kapillarrohrdurchgang (232) in mindestens einem von Durchmesser und Länge identisch oder voneinander verschieden sind und das mindestens eine von Durchmesser und Länge des ersten Kapillarrohrdurchgangs (222) oder des zweiten Kapillarrohrdurchgangs (232) eingestellt wird, um die Zufuhrmenge des flüssigen Medikaments zu regulieren.

8. Injektionsvorrichtung (100) für flüssige Medikamente, umfassend die Vorrichtung (200) nach einem der Ansprüche 1 bis 5.

## Revendications

1. Dispositif pour réguler la distribution d'un médicament liquide (200), comprenant :
un boîtier (201) ;
un corps (210) disposé à l'intérieur du boîtier (201), et possédant un orifice d'entrée (211) à travers lequel un médicament liquide provenant d'un tube d'entrée de médicament liquide (121a) s'étendant pour être en communication avec un espace de stockage de médicament liquide s'écoule dans le corps (210), et un orifice d'évacuation (212) pour évacuer le médicament liquide, qui s'est écoulé dans le corps (210) à travers l'orifice d'entrée (211), dans un tube de sortie de médicament liquide (121b) ;
un passage de débit d'alimentation continu (CL) situé à l'intérieur du corps (210) et configuré pour réguler un débit du médicament liquide s'écoulant dans le corps (210) à travers l'orifice d'entrée (211) de sorte qu'une certaine quantité de médicament liquide est continuellement fournie au tube de sortie de médicament liquide (121b) à travers l'orifice d'évacuation (212) du corps (210) ;
un passage d'écoulement de fourniture d'incrément (VL1, VL2) configuré pour diviser et stocker le médicament liquide s'écoulant dans le corps (210) à travers l'orifice d'entrée (211) du corps (210), puis pour permettre au médicament liquide divisé et stocké de rejoindre une extrémité de sortie du passage de débit d'alimentation continu (CL) de manière à augmenter la quantité de médicament liquide évacuée par l'orifice d'évacuation (212) du corps (210) ; et
un moyen d'élimination d'air (224, 225, 225a, 226) situé entre l'extrémité de sortie du passage de débit d'alimentation continu (CL) et l'orifice d'évacuation (212) du corps (210) et configuré pour évacuer l'air, qui est entraîné dans le médicament liquide passant à travers l'extrémité de sortie du passage de débit d'alimentation continu (CL) ou qui se trouve entre des parties du médicament liquide passant à travers l'extrémité de sortie du passage de débit d'alimentation continu (CL), vers l'extérieur du corps (210) de manière à éliminer l'air,
**caractérisé en ce que** le moyen d'élimination d'air (224, 225, 225a, 226) comprend une rainure (224) formée de manière concave sur un côté du corps (210) de manière à permettre à une partie du passage de débit d'alimentation continu (CL) d'être en communication avec l'extérieur du corps (210), un élément de couvercle (225) couplé à une partie d'ouverture de la rainure (224) de manière à fermer hermétiquement la rainure (224), au moins un évent (225a) formé dans l'élément de couvercle (225) de manière à évacuer vers l'extérieur l'air du médicament liquide passant à travers la rainure (224) dans le passage de débit d'alimentation continu (CL), et un élément filtrant (226) logé entre la rainure (224) et l'élément de couvercle (225) de manière à permettre à l'air dans le médicament liquide introduit dans la rainure (224) de passer à travers l'élément filtrant (226) et d'être évacué par l'évent (225a) et à ne pas permettre au médicament liquide de passer à travers l'élément filtrant (226), empêchant ainsi la fuite du médicament liquide.

2. Dispositif (200) selon la revendication 1, dans lequel le passage de débit d'alimentation continu (CL) comprend un passage de liaison (211a) qui est en communication avec l'orifice d'entrée (211) à l'intérieur du corps (210), un premier passage de tube capillaire (222) qui est en communication avec le passage de liaison (211a), et un premier passage de flux interne (223) pour relier le premier passage de tube capillaire (222) à l'orifice d'évacuation (212) à l'intérieur du corps (210) de façon à évacuer le médicament liquide, qui a traversé le premier passage de tube capillaire (222), à travers l'orifice d'évacuation (212).

3. Dispositif (200) selon la revendication 2, dans lequel le passage de flux de fourniture d'incrément (VL1, VL2) comprend un passage de flux interne (VL1) situé à l'intérieur du corps (210), et un passage de flux externe (VL2) situé à l'extérieur du corps (210) et qui est en communication avec le passage de flux interne (VL1).

4. Dispositif (200) selon la revendication 3, dans lequel le passage de flux interne (VL1) comprend un passage de liaison (211a) qui est en communication avec l'orifice d'entrée (211) à l'intérieur du corps (210), un second passage de tube capillaire (232) qui est en communication avec le passage de liaison (211a), un second passage de flux interne (233) qui est en communication avec le second passage de tube capillaire (232), et un orifice de sortie (234) du second passage de flux interne (233) pour guider le médicament liquide, qui a traversé le second passage de tube capillaire (232), vers le passage de flux externe (VL2).

5. Dispositif (200) selon la revendication 4, dans lequel le passage de flux externe (VL2) comprend un tube d'entrée de réservoir (301) pour relier l'orifice de sortie (234) du second passage de flux interne (233) à une entrée de réservoir à l'extérieur du corps (210), un réservoir (300) possédant un espace de stockage de médicament liquide (302) pour stocker le médicament liquide fourni à travers le tube d'entrée de réservoir (301), et un tube de sortie de réservoir (303) pour permettre au médicament liquide, qui est évacué de l'espace de stockage de médicament liquide (302) du réservoir (300) à travers une sortie de réservoir, de rejoindre le premier passage de flux interne (223).

6. Dispositif (200) selon la revendication 1, dans lequel l'élément filtrant (226) se présente sous la forme d'un filtre double comprenant une couche filtrante hydrophile (226a) et une couche filtrante hydrophobe perméable aux gaz et imperméable aux liquides (226b) empilée sur la couche filtrante hydrophile (226a).

7. Dispositif (200) selon la revendication 4 ou 5, dans lequel le premier passage de tube capillaire (222) et le second passage de tube capillaire (232) sont identiques ou différents l'un de l'autre dans au moins l'un parmi un diamètre ou une longueur, et l'au moins un parmi le diamètre ou la longueur du premier passage de tube capillaire (222) ou du second passage de tube capillaire (232) est ajusté pour réguler la quantité de distribution du médicament liquide.

8. Appareil d'injection de médicament liquide (100) comprenant le dispositif (200) selon l'une quelconque des revendications 1 à 5.
